# EUROPÄISCHE PATENTSCHRIFT

(11) **EP 2 735 384 B1**
(45) Veröffentlichungstag und Bekanntmachung des Hinweises auf die Patenterteilung: **28.08.2019**
(21) Anmeldenummer: 13174247.0
(22) Anmeldetag: 15.06.2011
(51) Int. Cl.: B21C 37/02, B21K 23/00, A61K 6/04, B21H 8/00

(54) **Barren aus Edelmetall und Verfahren zur Herstellung**
Ingots made of precious metal and production method thereof
Barres en métal précieux et leur procédé de fabrication

(30) Priorität: 15.06.2010 DE 102010030128; 20.11.2010 DE 102010044199
(43) Veröffentlichungstag der Anmeldung: 28.05.2014
(62) Teilanmeldung aus: 11731292.6
(73) Patentinhaber: ESG Edelmetall-Service GmbH&Co. KG, 76287 Rheinstetten (DE)
(72) Erfinder: Lochmann, Dominik, 76287 Rheinstetten (DE)
(74) Vertreter: Sommer, Peter

(56) Entgegenhaltungen:
- FR-A- 348 205
- FR-A- 1 152 976
- JP-A- 1 133 395
- JP-A- 3 019 393

## Beschreibung

Die Erfindung betrifft einen Barren aus Edelmetall, beispielsweise Gold, Silber, Platin, Palladium oder Legierungen davon und ein Verfahren zur Herstellung eines solchen Barren (siehe z.B. WO 00/16932 oder DE 2 107 213).

### Stand der Technik

Dosierbare Zahngoldmaterialien sind im Handel als Plättchen erhältlich, wobei die Plättchen durch Herstellung von identischen Abschnitten aus einem gewalzten Goldblech erhalten wurden. Das Goldblech wird durch Auswalzen eines legierten Goldbarrens bereitgestellt, so beschrieben in der DE 10 2004 060 730 A1.

Bei der Herstellung von Goldbarren in kleiner Stückelung, also 1 Gramm, 5 Gramm, 10 Gramm ist es bekannt, auf jedem einzelnen Barren das Gewicht, der Hersteller, die Reinheit des Metalls und das Metall einzuprägen. Barren aus Edelmetall in kleiner Stückelung sind bei Anlegern zwar sehr beliebt, einzeln in der Herstellung aber im Verhältnis zum Metallpreis teuer.

### Darstellung der Erfindung

Der Grundgedanke der Erfindung besteht darin, anstelle vieler einzelner Kleinbarren mit jeweils einer vorgegebenen Masse mk, mehrere solcher Kleinbarren aus einer Tafel oder aus einem Barren mit einer Masse mB auf einmal herzustellen und diese Tafel oder diesen Barren dann zur Ausbildung der Kleinbarren ganz oder teilweise stofflich von einander zu trennen, wobei die Kleinbarren in einer n x m-Anordnung mit n, m ≥ 2 angeordnet sind. Insgesamt ergibt sich also wegen n x m x mk die Masse mB des Barrens.

Dadurch, dass zunächst ein Barren in Form einer Tafel mit einer Sollmasse mB hergestellt wird, der dann, beispielsweise durch Prägen, in viele Kleinbarren aufgeteilt wird, die im Bedarfsfall ohne Verwendung von Werkzeug von einander abzutrennen bzw. von einem Träger zu lösen sind, lassen sich sowohl die Herstellungskosten verringern als auch die Handhabung der Kleinbarren verbessern.

Die Erfindung betrifft daher einen Barren aus Edelmetall oder einer Edelmetall aufweisenden Legierung mit einer Masse mB, wobei der Barren in n x m Kleinbarren mit jeweils einer vorgegebenen Masse mk unterteilt ist, wobei n, m jeweils eine natürliche Zahl ≥ 2 ist. Zwischen den unmittelbar benachbarten Kleinbarren besteht eine stoffliche Verbindung, sodass die Kleinbarren mit ihren unmittelbaren Nachbarn fest verbunden sind. Die stoffliche Verbindung ist Teil einer in den Barren eingebrachten zulaufen ausgebildeten Vertiefung und kann beispielsweise in Form einer Brücke oder als Verbindungssteg ausgebildet sein.

Die stoffliche Verbindung weist in der größten Tiefe der Vertiefung eine Sollbruchstelle auf, die ein Abtrennen von Hand ermöglicht. Dabei ist die Biegefestigkeit der Verbindung vorzugsweise so groß, dass ein Verbiegen unter Einwirkung der Schwerkraft vermieden wird und höchstens so groß, dass ein Zerstören der stofflichen Verbindung von Hand durch Biegen oder Brechen möglich ist. Die Sollbruchstelle weist im Bereich der stofflichen Verbindung einen Öffnungswinkel von 10° bis 60° auf.

Zusätzlich zu der stofflichen Verbindung kann auf einer Unterseite des Barren ein Trägermaterial angebracht sein.

Dabei kann eine Vertiefung auf einer Oberseite und eine Vertiefung gegenüberliegend auf einer Unterseite ausgebildet sein und die stoffliche Verbindung kann zu der Oberseite und zu der Unterseite des Barrens beabstandet sein. Die Vertiefung kann als Rille ausgebildet sein.

Ein weiterer Gegenstand der Erfindung ist ein Verfahren zur Herstellung eines Barren mit einer Masse mB aus Edelmetall oder einer Edelmetall aufweisenden Legierung, bei dem der Barren in einem Herstellungsschritt in n x m Kleinbarren mit jeweils einer vorgegebenen Masse mk unterteilt wird, wobei n, m jeweils eine natürliche Zahl ≥ 2 ist, wobei zwischen den unmittelbar benachbarten Kleinbarren eine stoffliche Verbindung mit einer Sollbruchstelle bestehen bleibt.

Die stoffliche Verbindung kann als Teil einer Vertiefung ausgebildet sein.

Vorteilhafterweise kann der Barren zur Aufteilung in Kleinbarren geprägt sein.

Noch ein weiterer Gegenstand der Erfindung ist ein Verfahren zur Herstellung eines Barren mit einer Masse mB aus Edelmetall oder einer Edelmetall aufweisenden Legierung, bei dem ein Edelmetall-Endlosband in einer entsprechenden Dicke gewalzt wird und einer Umformeinrichtung schrittweise zugeführt und nach einer Umformung das Band weitergeschoben wird. Bei der Umformung wird das Endlosband in eine Reihe aus n x 1 Kleinbarren mit jeweils einer vorgegebenen Masse mk unterteilt, wobei n eine natürliche Zahl ≥ 2 ist und wobei zwischen unmittelbar benachbarten Kleinbarren und dem Endlosband eine stoffliche Verbindung mit einer Sollbruchstelle bestehen bleibt. Auf diese Weise entsteht ein Endlosverbundbarren.

Wenn das umgeformte Endlosband zur Herstellung des Barrens nach einer vorgegebenen Zahl von Reihen im Bereich der stofflichen Verbindung zwischen einer Reihe und dem Endlosband getrennt wird, kann der gewünschte Barren entstehen.

Das Endlosband weist eine Breite B größer als die Breite b des herzustellenden Barrens auf, sodass beim Umformen des Endlosbandes zusätzlich zu dem Barren ein überstehender Rand entsteht.

Wenn bei der Umformung ein automatisches Abtrennen des Randes erfolgt, kann nach einer Herstellung einer gewünschten Zahl von Reihen eine Abtrennung vom Endlosband erfolgen und der fertige Barren liegt dann vor.

Vorteilhafterweise kann der beim Umformen entstehende Kleinbarren gleichzeitig beschriftet werden.

### Kurzbeschreibung der Zeichnung

In der Zeichnung sind Ausführungsbeispiele der Erfindung dargestellt. Es zeigt:
- Fig. 1: einen Barren aus Edelmetall in Form einer Tafel mit 4 x 3 Kleinbarren;
- Fig. 2: ein Detail aus Fig. 1;
- Fig. 3: einen Teilschnitt entlang der Linie AA aus Fig. 2;
- Fig. 4A bis 4B: verschiedene Formen einer stofflichen Verbindung zwischen den Kleinbarren einer Tafel;
- Fig. 5A bis 5B: weitere Formen einer stofflichen Verbindung zwischen den Kleinbarren einer Tafel;
- Fig. 6: ein im Schnitt dargestelltes Endlosband mit Kleinbarren;
- Fig. 7: eine Draufsicht auf das umgeformte Endlosband aus Fig. 6;
- Fig. 8: Kanten eines Werkzeugs für die Herstellung von Kleinbarren.

### Ausführungsbeispiele der Erfindung

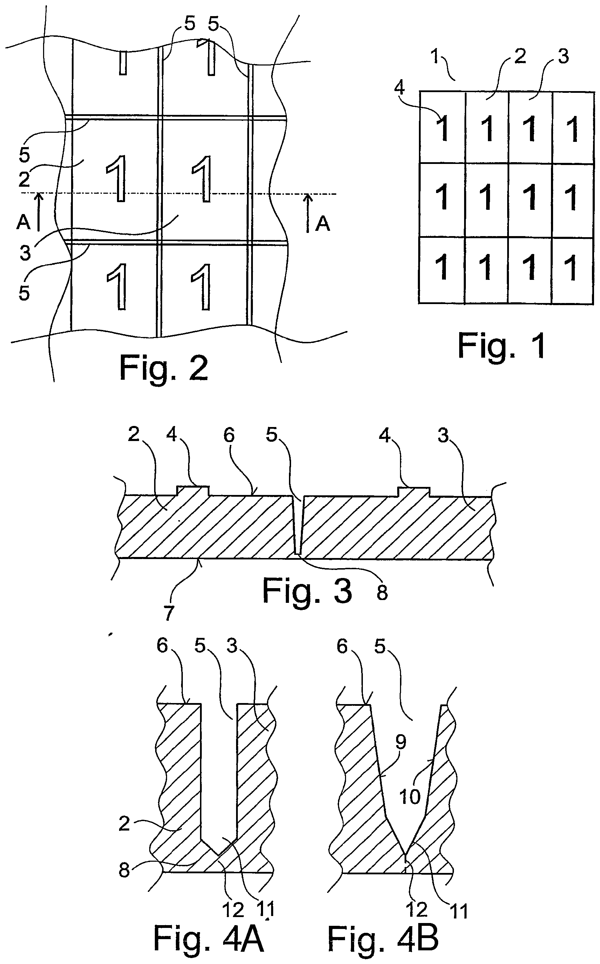

In Fig. 1 ist ein Barren 1 aus Edelmetall in Form einer Tafel mit 4 x 3 Kleinbarren 2,3 gezeigt. Dafür wurde zunächst ein nicht dargestellter Barren in Form einer Tafel mit einer Sollmasse mB hergestellt. Die Bearbeitung des Barrens zur Bereitstellung vieler Kleinbarren 2,3 kann durch Umformen wie beispielsweise Prägen erfolgen, und zwar bereits mit einer Umformung wie einem Prägevorgang, bei dem auch die Angaben 4 über den Kleinbarren 2,3 erzeugt werden, nämlich ein Herstellerlogo, die Masse und die Reinheit, so dass dann die Herstellung einem einzigen Arbeitsgang erfolgen kann. Im Ausführungsbeispiel ist als Angabe 4 die Masse mit 1 angegeben, was 1g bedeutet.

Wie in Fig. 2 im Detail dargestellt, wurden durch den Umformvorgang in den Barren 1 in vorgegebenen Abständen Vertiefungen in Form von Rillen 5 eingebracht, die einen einzelnen Kleinbarren 2 von dem benachbarten Kleinbarren 3 klar erkennbar abgrenzen. Die Lage der Rillen 5 in dem Barren 1 ist so gewählt, dass die durch die Rillen 5 begrenzten Kleinbarren 2,3 die gewünschte Masse aufweisen.

Der Barren kann vor dem Umformvorgang vorzugsweise eine vorgegebene gleichmäßige Dicke aufweisen, damit die Umformung des Barrens mit einem einzigen Prägewerkzeug vorgenommen werden kann und die Masse für die Kleinbarren 2,3 genau genug, also innerhalb der zulässigen Toleranzen, erreicht wird.

Wie ein Barren als Tafel hergestellt werden kann, wird am Beispiel eines Feingoldbarrens mit einer Masse von 100 g erläutert, der in 100 Kleinbarren zu je 1 g unterteilt wird. Dazu wird ein Endlosblechband aus 99,99% Feingold auf eine vorher berechnete Dicke ausgewalzt. Aus diesem Band werden Tafeln von je 100g ausgestanzt.

Diese Tafeln werden in einer für die Prägung von Münzen in ähnlicher Weise bereits bekannten Prägemaschine in einem Arbeitsschritt so geprägt, dass Vertiefungen in Form von Rillen 5 zwischen den einzelnen Kleinbarren entstehen, und dass auf jedem einzelnen 1 g Kleinbarren das Herstellerlogo, das Gewicht und die Reinheit eingeprägt werden.

Die als Rillen ausgebildeten Vertiefungen 5, 5' können so dünn ausgeführt werden, dass das verdrängte Material nur einen vergleichsweise kleinen seitlichen Wulst ergibt, welcher durch den Prägevorgang der Flächen aber sofort wieder abgeflacht werden kann, wenn es gewünscht ist. Dünn bedeutet in diesem Zusammenhang, dass die Breite der Vertiefung geringer als deren Tiefe ist und vorzugsweise höchstens 50% der Tiefe beträgt.

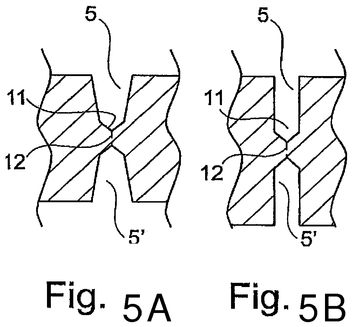

Fig. 3 zeigt einen Teilschnitt entlang der Linie AA aus Fig. 2 und es wird erkennbar, dass die Vertiefungen 5 nicht durch die ganze Tafel hindurch gehen, sondern dass eine von einer Oberseite 6 in Richtung zu einer Unterseite 7 hin ausgehende Rille 5 ausgebildet ist, wobei die stoffliche Verbindung 8 in Form eines Verbindungsstegs, siehe später Fig. 4A-4B, oder einer Brücke, siehe später Fig. 5A-5B besteht.

Die stoffliche Verbindung 8 in der Vertiefung 5 kann dabei in unterschiedlicher Weise ausgebildet sein, wie in den Fig. 4A-4B und 5A-5B beispielhaft gezeigt ist.

In Fig. 4A sind Seitenwände 9,10 der nur von der Oberseite der Tafel her eingebrachten Vertiefung 5 im wesentlichen parallel zueinander und quer zur Oberseite 6 und der Unterseite 7 der Tafel. Der Boden 11 ist dabei Teil des Verbindungsstegs der stofflichen Verbindung 8 der benachbarten Kleinbarren 2,3 untereinander.

Der Boden 11 der Vertiefung ist zur Unterseite hin zulaufend ausgebildet, sodass in der größten Tiefe des Bodens 11 wegen des geringsten Querschnitts des Verbindungsstegs eine Sollbruchstelle 12 ausgebildet ist.

Mit einer Sollbruchstelle 12 lässt sich die stoffliche Verbindung 8 in der Vertiefung 5 im Bedarfsfall ohne Verwendung von Werkzeug lösen und die Kleinbarren können von einander abgetrennt werden. Dabei wird aufgrund der bekannten Lage der Sollbruchstelle eine planmäßige Verteilung der Massen sichergestellt.

In Fig. 4B sind die Seitenwände der nur von der Oberseite 6 der Tafel her eingebrachten Vertiefung 5 zum Boden hin schräg zulaufend ausgebildet, wobei der Boden 11 der Vertiefung 5 wie in Fig. 4B spitz zulaufend ausgebildet ist. Dadurch ergibt sich eine Sollbruchstelle 12. Im Querschnitt ergibt sich ein Verlauf mit zwei unterschiedlichen Öffnungswinkeln der Vertiefung, wobei der Öffnungswinkel des Bodens 11 größer ist als der Öffnungswinkel der Seitenwände 9,10.

In den Fig. 5A-5B sind weitere Formen einer stofflichen Verbindung 8 zum Teil mit Sollbruchstelle 12 zwischen den Kleinbarren 2,3 einer Tafel dargestellt, die sich von denen der Fig. 4A-4B dadurch unterscheiden, dass die stoffliche Verbindung in einer sowohl von der Oberseite 6 als auch von der Unterseite 7 eingebrachten Vertiefung 5,5' angeordnet ist. In diesem Fall wird das Material sowohl an die Oberseite 6 als auch an die Unterseite 7 verdrängt und kann, falls gewünscht, im Prägevorgang abgeflacht werden.

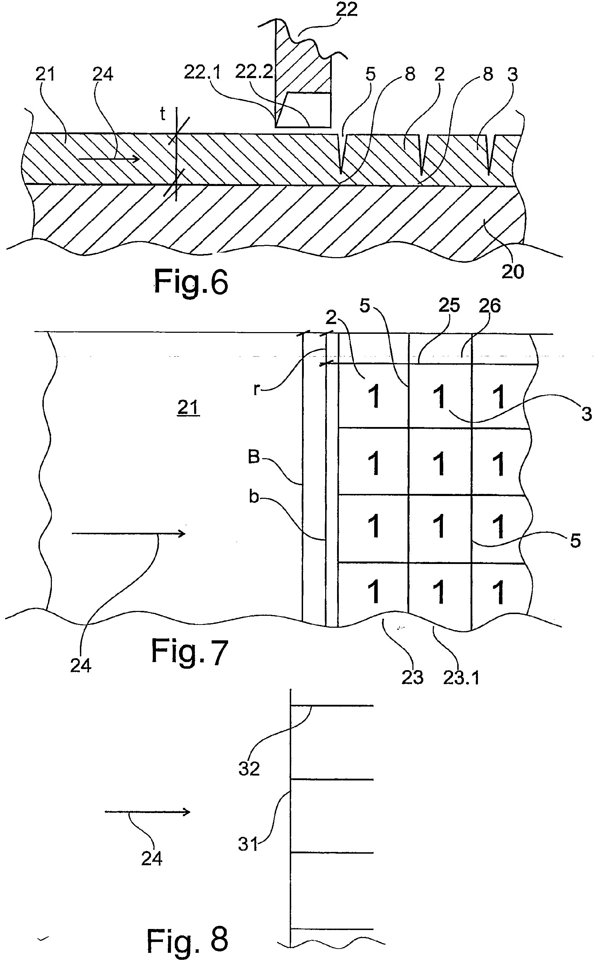

In einer weiteren, in den Fig. 6-8 dargestellten Ausführungsform wird der Barren 1 so hergestellt, dass ausgehend von einem in Fig. 6 im Schnitt dargestellten, auf eine vorberechnete Dicke t ausgewalztes Endlosband 21 aus 99,99% Feingold eine schrittweise Umformung des Endlosbandes 21 unter Ausbildung der Kleinbarren erfolgt. Dabei wird das auf einem Tisch 20 aufliegende Endlosblechband 21 an eine Umformstation 22 herangeführt und in einem Umformschritt werden Kleinbarren 2 erzeugt, die mit einem im vorherigen Umformschritt erzeugten Kleinbarren 3 und zusätzlich auch noch mit dem noch nicht umgeformten Endlosband 21 jeweils am Boden einer als Rille ausgebildete Vertiefung 5 eine stoffliche Verbindung 8 aufweisen. Die als Rille ausgebildete Vertiefung 5 wird mit einer scharfen Kante 22.1 erzeugt, eine weitere scharfe Kante 22.2 ist für nicht dargestellte Rillen in Zufuhrrichtung 24 gezeigt.

Wie aus der Draufsicht aus Fig. 7 ersichtlich ist, sind die Kleinbarren 2, 3 in jeweils einer Reihe 23, 23.1. quer zu der Zufuhrrichtung 24 des Endlosbandes 21 angeordnet und durch die als Rille ausgebildete Vertiefung 5 voneinander abgesetzt.

Ist die Umformung einer Reihe 23 abgeschlossen, wird das Endlosblechband 21 gegenüber der nicht dargestellten Umfor meinrichtung einen Schritt weiter vorgeschoben und es findet eine erneute Umformung statt, um die nächste Reihe von Kleinbarren 2, 3 zu erzeugen. Der Barren selbst kann dann durch geeignetes Abtrennen auf die gewünschte Anzahl von Reihen 23 von Kleinbarren 2, 3 gebracht werden.

Wenn das Endlosblechband 21 eine Breite B größer als die Breite b des herzustellenden Barren aufweist, wird jeder Kleinbarren 2, 3 bei seiner Herstellung denselben Umformungen unterworfen. Allerdings entsteht durch das seitliche Übermaß des Endlosblechbandes 21 gegenüber dem Barren 1 dann ein durch eine als Rille ausgebildete Vertiefung 25 abgesetzter aber gleichwohl noch stofflich verbundener seitlicher Rand 26 der Breite r, der sich in Zufuhrrichtung 25 erstreckt. Der Rand 26 kann bereits beim Umformen abgetrennt werden oder er wird erst nach dem Umformen abgetrennt.

Auch bei dieser Umformung kann die Erzeugung von Vertiefungen 5 sowohl von der Oberseite her als auch von der Unterseite her des Endlosbandes 21 erfolgen. Die zusätzliche Umformung von der Unterseite her ist grundsätzlich dann vorteilhaft, wenn die Dicke des Barrens so groß ist, dass die Umformung von nur einer Seite her nicht ausreicht.

Die durch die Einbringung von Vertiefungen 5 in den Barren erzeugten Sollbruchstellen im Bereich der stofflichen Verbindung 8 können einen Öffnungswinkel von etwa 10° bis 60° aufweisen und die stoffliche Verbindung 8 kann eine Dicke von 0,05mm bis 0,4mm aufweisen, wobei auch andere Dicken noch ein Abtrennen von Hand ermöglichen.

In Fig. 8 sind Kanten 31, 32 eines Werkzeugs für die Herstellung von Kleinbarren dargestellt. Eine Kante 31 verläuft quer zur Zuführrichtung 24 und erzeugt die als Rille ausgebildete Vertiefung 5, eine weitere Kante 32 in Zuführrichtung 24 erzeugt beispielsweise die als Rille ausgebildete Vertiefung 25 am Rand 26 eines Kleinbarrens 2, 3 aus Fig. 7. Die Kanten 231, 32 werden in das Endlosband gedrückt und verdrängen das Material, sodass eine Vertiefung entsteht, wobei gleichzeitig eine stoffliche Verbindung bestehen bleibt, was hier nicht dargestellt ist.

Auch bei der Ausführungsform nach den Fig. 6-8 ist es möglich, anstelle einer stofflichen Verbindung ein Träger vorzusehen, der mit dem Endlosband verbunden ist.

Die Barren werden beispielsweise in den nachfolgend angegebenen Größen hergestellt. Für Gold 100x1g: 74mm x 105mm x 0,667mm oder 85mm x 150mm x 0,406mm; für Silber: 100x1g: 74mm x 105mm x 1,226mm; für Platin: 100x1g: 74mm x 105mm x 0,602mm und für Palladium: 100x1g : 74mm x 105mm x 1,073mm.

Es lassen sich hier also zwei Herstellungsvarianten unterscheiden, wobei andere Herstellungsverfahren, etwa durch Gießen, nicht ausgeschlossen werden. In der ersten Variante wird ein Edelmetallblech auf die fertigen Endmaße geschnitten. Das zugeschnittene Blech kommt dann in eine normale Prägemaschine, wie sie auch für Münzen oder normale Edelmetallbarren eingesetzt wird und wird dort mit entsprechend ausgeformten Prägestempeln unter hohem Druck in die Endform geprägt.

In der zweiten Variante wird ein Edelmetall-Endlosband in entsprechender Dicke gewalzt und in einer Stanzmaschine jeweils eine komplette Reihe, etwa 10 Kleinbarren zu 1g gleichzeitig gekerbt und beschriftet. Danach wird das Band weitergeschoben, so dass ein Endlosverbund entsteht, der dann jeweils nach 10 Reihen gekappt werden kann, um einen Barrenverbund in einer Stückelung von 10x10x1g zu erhalten.

Mit beiden Varianten können auch hochglänzende Oberfläche hergestellt werden.

## Patentansprüche

1. Barren aus Edelmetall oder einer Edelmetall aufweisenden Legierung, mit einer Masse mB, **dadurch gekennzeichnet, dass** der Barren (1) in n x m Kleinbarren (2,3) mit jeweils einer vorgegebenen Masse mk unterteilt ist, wobei n, m jeweils eine natürliche Zahl ≥ 2 ist, dass zwischen den unmittelbar benachbarten Kleinbarren (2,3) eine stoffliche Verbindung (8) besteht, die Teil einer in den Barren (1) eingebrachten, zulaufend ausgebildeten Vertiefung (5,5') ist und die in der größten Tiefe der Vertiefung (5, 5') eine Sollbruchstelle (12) mit einer Dicke aufweist, die ein Abtrennen von Hand ermöglicht, wobei die Sollbruchstelle (12) im Bereich der stofflichen Verbindung (8) einen Öffnungswinkel von 10° bis 60° aufweist.

2. Barren nach Anspruch 1, **dadurch gekennzeichnet, dass** die Vertiefungen in Form von Rillen ausgebildet und in vorgegebenen Abständen eingebracht sind und dass die Lage der Rillen in dem Barren so gewählt ist, dass die durch die Rillen begrenzten Kleinbarren (2, 3) die gewünschte Masse mk aufweisen.

3. Barren nach einem der Ansprüche 1 bis 2, **dadurch gekennzeichnet, dass** eine Vertiefung (5) auf einer Oberseite (6) und eine Vertiefung (5') gegenüberliegend auf einer Unterseite (7) ausgebildet ist und dass die stoffliche Verbindung (8) zu der Oberseite (6) und zu der Unterseite (7) des Barrens beabstandet ist.

4. Barren nach einem der Ansprüche 1 bis 3, **dadurch gekennzeichnet, dass** der Barren als die fertigen Endmaße aufweisende Tafel mit einer Masse mB hergestellt ist, und dass in diese Tafel die Vertiefungen eingeprägt sind.

5. Verfahren zur Herstellung eines Barren mit einer Masse mB aus Edelmetall oder einer Edelmetall aufweisenden Legierung, **dadurch gekennzeichnet, dass** der Barren (1) in einem Herstellungsschritt in n x m Kleinbarren (2,3) mit jeweils einer vorgegebenen Masse mk unterteilt wird, wobei n, m jeweils eine natürliche Zahl ≥ 2 ist, wobei zwischen den unmittelbar benachbarten Kleinbarren (2,3) eine stoffliche Verbindung (8) mit einer Sollbruchstelle (12) mit einer Dicke bestehen bleibt, die ein Abtrennen von Hand ermöglicht.

6. Verfahren zur Herstellung eines Barren mit einer Masse mB aus Edelmetall oder einer Edelmetall aufweisenden Legierung, bei dem ein Edelmetall-Endlosband in einer entsprechenden Dicke gewalzt wird und einer Umformeinrichtung schrittweise zugeführt und nach einer Umformung das Band weitergeschoben wird, **dadurch gekennzeichnet, dass** bei der Umformung das Endlosband in eine Reihe (23; 23.1) aus n x 1 Kleinbarren (2; 3) mit jeweils einer vorgegebenen Masse mk unterteilt wird, wobei n eine natürliche Zahl ≥ 2 ist, wobei zwischen unmittelbar benachbarten Kleinbarren (2, 3) und dem Endlosband (21) eine stoffliche Verbindung (8) mit einer Sollbruchstelle (12) mit einer Dicke bestehen bleibt, die ein Abtrennen von Hand ermöglicht, wobei das Endlosband (21) eine Breite B größer als die Breite b des herzustellenden Barrens (1) aufweist, sodass beim Umformen zusätzlich zu dem Barren (1) ein überstehender Rand (26) entsteht.

7. Verfahren nach Anspruch 6, **dadurch gekennzeichnet, dass** das umgeformte Endlosband zur Herstellung des Barrens nach einer vorgegebenen Zahl von Reihen (23, 23.1) im Bereich der stofflichen Verbindung (8) zwischen einer Reihe (23) und dem Endlosband (21) getrennt wird.

8. Verfahren nach einem der Ansprüche 5 bis 7, **dadurch gekennzeichnet, dass** der Kleinbarren bei seiner Herstellung gleichzeitig beschriftet wird.

9. Verfahren nach einem der Ansprüche 5 bis 8, **dadurch gekennzeichnet, dass** bei der Unterteilung des Barrens in Kleinbarren die stoffliche Verbindung als Teil einer Vertiefung in den Barren eingebracht wird.

10. Verfahren nach Anspruch 9, **dadurch gekennzeichnet, dass** die durch Einbringung von Vertiefungen erzeugten Sollbruchstellen im Bereich der stofflichen Verbindung (8) mit einem Öffnungswinkel von 10° bis 60° ausgebildet werden.

11. Verfahren nach einem der Ansprüche 5 bis 10, **dadurch gekennzeichnet, dass** die stoffliche Verbindung (8) mit einer Dicke von 0,05 mm bis 0,4 mm hergestellt wird.

12. Verfahren nach einem der Ansprüche 8 bis 11, **dadurch gekennzeichnet, dass** der Barren durch Stanzen hergestellt wird.

13. Verfahren nach Anspruch 5, **dadurch gekennzeichnet, dass** der Barren durch Gießen hergestellt wird.

## Claims

1. A bar of noble metal or an alloy containing a noble metal, having a mass mB, **characterized in that** said bar (1) is subdivided into n x m miniature bars (2, 3) each having a specified mass mk, n and m each denoting an integer ≥ 2, and that an interconnection of solid material (8) is provided between the directly adjacent miniature bars (2, 3) said interconnection of solid material (8) being part of a tapered depression formed in said bar (1) and that said interconnection of solid material (8) has a break point (12) in the deepest region of said depression(5,5') and has a thickness enabling manual break-off thereof, the said break point (12) having in the region of said solid material (8) an aperture angle of 10° to 60°.

2. The bar as defined in claim 1, **characterized in that** said depressions are in the form of grooves disposed at specific distances, the position of said grooves in said bar being such that the miniature bars (2, 3) delimited by said grooves have the desired mass mk.

3. The bar as defined in any one of claims 1 to 2, **characterized in that** a depression (5) is formed on the top surface (6) and a depression (5') is formed oppositely thereto on the underside (7), and that said interconnection of solid material (8) is set at a distance from said top surface (6) and from said underside (7) of said bar.

4. The bar as defined in any one of claims 1 to 3, **characterized in that** said bar is manufactured as a tablet having the desired size and a mass mB and that said depressions are impressed into said tablet.

5. A method for the production of a bar having a mass mB consisting of noble metal or an alloy containing noble metal, **characterized in that,** in one manufacturing step, the bar (1) is subdivided into n x m miniature bars (2, 3) each having a specified mass mk, n and m each denoting an integer ≥ 2, and said interconnection of solid material (8) remains intact between the directly adjacent miniature bars (2, 3), and has a break point (12) and a thickness enabling manual break-off thereof.

6. A method for the production of a bar having a mass mB of noble metal or an alloy containing noble metal in which a continuous band of noble metal is rolled out to a desired thickness and is fed stepwise to a shaping device and moved away therefrom following shaping, **characterized in that** during said shaping said continuous band is subdivided into a row (23, 23.1) of n x 1 miniature bars (2, 3) of which each has a specified mass mk, in which n denotes an integer ≥ 2, an interconnection of solid material (8) being left intact between the directly adjacent miniature bars (2, 3) and having a break point (12) and a thickness enabling manual break-off thereof, the said continuous band (21) having a width B that is greater than the width b of the bar (1) to be fabricated such that shaping produces said bar (1) and additionally a protruding edge (26).

7. The method as defined in claim 6, **characterized in that**, during the production of said bar, the shaped continuous band is severed in the region of said interconnection of solid material (8) between a row (23) and said continuous band (21) following the creation of a specified number of rows (23, 23.1).

8. The method as defined in any one of claims 5 to 7 **characterized in that** said miniature bar is inscribed during production thereof.

9. The method as defined in any one of claims 5 to 8, **characterized in that** said interconnection of solid material is introduced into said bar in the form of a depression when the bar is subdivided into miniature bars.

10. The method as defined in claim 9, **characterized in that** the break points introduced by the introduction of depressions are formed in the region of the said interconnection of solid material (8) so as to have an aperture angle of from 10° to 60°.

11. The method as defined in any one of claims 5 to 10, **characterized in that** said interconnection of solid material (8) is produced with a thickness of from 0.05 mm to 0.4 mm.

12. The method as defined in any one of claims 5 to 11, **characterized in that** said bar is produced by stamping.

13. The method as defined in claim 5, **characterized in that** said bar is produced by casting.

## Revendications

1. Lingot en métal précieux ou en un alliage comprenant un métal précieux, d'une masse mB, **caractérisé en ce que** le lingot (1) est divisé en n x m mini-lingots (2, 3) ayant chacun une masse mk prédéfinie n, m étant chacun un entier naturel ≥ 2, **en ce qu'**entre les mini-lingots (2, 3) directement voisines est présente une liaison par matière (8) qui est une partie d'un creux (5, 5') ménagé dans la barre (1) conçu en pointe et qui dans la plus grande profondeur du creux (5, 5') comporte une zone de rupture théorique (12) d'une épaisseur qui permet un sectionnement manuel, dans la zone de la liaison par matière (8), la zone de rupture théorique (12) présentant un angle d'ouverture de 10° à 60°.

2. Lingot selon la revendication 1, **caractérisé en ce que** les creux sont conçus sous la forme de cannelures et sont ménagés avec des écarts prédéfinis et **en ce que** l'emplacement des cannelures dans le lingot est sélectionné de telle sorte que les mini-lingots (2, 3) délimités par les cannelures présentent la masse mk.

3. Lingot selon l'une quelconque des revendications 1 à 2, **caractérisé en ce qu'**un creux (5) est conçu sur une face supérieure (6) et un creux (5') est conçu en vis-à-vis sur une face inférieure (7) et **en ce que** la liaison par matière (8) est écartée de la face supérieure (6) et de la face inférieure (7) du lingot.

4. Lingot selon l'une quelconque des revendications 1 à 3, **caractérisé en ce que** le lingot est fabriqué comme une tablette présentant les cotes finies avec une masse mB et **en ce que** les creux sont gaufrés dans ladite tablette.

5. Procédé, destiné à produire un lingot d'une masse mB en métal précieux ou en un alliage comprenant un métal précieux, **caractérisé en ce que** dans une étape de procédé, le lingot (1) est divisé en n x m mini-lingots (2, 3), ayant chacun une masse mk prédéfinie, n, m étant chacun un entier naturel ≥ 2, entre les mini-lingots (2, 3) directement voisins étant conservée une liaison par matière (8) pourvue d'une zone de rupture théorique (12) d'une épaisseur qui permet un sectionnement manuel.

6. Procédé, destiné à produire un lingot d'une masse mB en métal précieux ou en un alliage comprenant un métal précieux, lors duquel la bande continue de métal précieux est laminée dans une épaisseur correspondante et est amenée progressivement vers un système de façonnage, et après un façonnage, la bande est poussée plus en avant, **caractérisé en ce que** lors du façonnage, la bande continue est divisée en une rangée (23 ; 23.1) de n x 1 mini-lingots (2 ; 3) ayant chacun une masse mk prédéfinie, n étant un entier naturel ≥ 2, entre deux mini-lingots (2, 3) directement voisins et la bande continue (21) étant conservée une liaison par matière (8) pourvue d'une zone de rupture théorique (12) d'une épaisseur qui permet un sectionnement manuel, la bande continue (21) ayant une largeur B supérieure à la largeur b du lingot (1) qui doit être produit, de telle sorte que lors du façonnage, en supplément du lingot (1), il soit donné naissance à un bord (26) débordant.

7. Procédé selon la revendication 6, **caractérisé en ce que** pour produire le lingot, la bande continue façonnée est sectionnée selon un nombre prédéfini de rangées (23, 23.1) dans la zone de la liaison par matière (8) entre une rangée (23) et la bande continue (21) .

8. Procédé selon l'une quelconque des revendications 5 à 7, **caractérisé en ce que** lors de sa production, le mini-lingot est simultanément muni d'une inscription.

9. Procédé selon l'une quelconque des revendications 5 à 8, **caractérisé en ce que** lors de la division du lingot en mini-lingots, la liaison par matière est ménagée dans le lingot, en tant que partie d'un creux.

10. Procédé selon la revendication 9, **caractérisé en ce que** les zones de rupture théorique créées par ménagement de creux dans la zone de liaison par matière (8) sont conçues avec un angle d'ouverture de 10° à 60°.

11. Procédé selon l'une quelconque des revendications 5 à 10, **caractérisé en ce que** la liaison par matière (8) est créée avec une épaisseur de 0,05 mm à 0,4 mm.

12. Procédé selon l'une quelconque des revendications 8 à 11, **caractérisé en ce que** le lingot est produit par estampage.

13. Procédé selon la revendication 5, **caractérisé en ce que** le lingot est produit par coulée.
